# EUROPEAN PATENT APPLICATION

(11) **EP 1 055 405 A1**
(43) Date of publication of application: **29.11.2000**
(21) Application number: 99959882.4
(22) Date of filing: 17.12.1999
(51) Int. Cl.: A61K 7/00

(54) **COSMETIC PREPARATION**

(30) Priority: 18.12.1998 JP 36136698
(71) Applicant: Kao Corporation, Tokyo 103-8210 (JP)
(72) Inventor: KAJINO, Takayoshi, Kao Corp. Res. Lab., Sumida-ku, Tokyo 131-8501 (JP); KAMIYA, Tetsuro, Kao Corp. Res. Lab., Sumida-ku, Tokyo 131-8501 (JP)
(74) Representative: HOFFMANN - EITLE
(86) International application number: JP9907105
(87) International publication number: WO0037028

(57) **Abstract**

A cosmetic composition contains (A) a quaternary ammonium base-containing cationic crosslinked copolymer, (B) powder having an average particle size of 0.05 to 50 µm, and (C) a water- compatible solvent. The composition is easy to apply to the skin, has a good feel and is excellent in dispersibility of powder.

## Description

### TECHNICAL FIELD

The present invention relates to a powder-containing cosmetic composition which is excellent in dispersibility of powder and has a good feel to the skin.

### BACKGROUND ART

Cosmetic compositions of a lotion type generally contain a water-compatible solvent such as ethanol. When powder is contained therein, however, it is difficult to stably disperse the powder therein. The viscosity of the system is preferably as high as possible for the purpose of retaining the dispersion stability. However, the feel of such a viscous cosmetic composition when applied to the skin or hair is impaired. Cosmetic compositions of a two-layer separation type in which the viscosity of the system is kept low, and the system is separated into two layers before use and made a uniform dispersion system upon use by shaking have also been known. However, these compositions have involved a problem that when the shaking is conducted after stored for a long period of time, powder precipitated becomes lumps and is hard to be dispersed again.

It is an object of the present invention to provide a cosmetic composition which is excellent in dispersibility of powder and has a good feel upon its application.

### DISCLOSURE OF THE INVENTION

The present inventors have found that when a quaternary ammonium base-containing cationic crosslinked copolymer is used in combination with powder having a specific particle size and a water-compatible solvent, a cosmetic composition which is excellent in dispersibility or redispersibility of the powder and has a good feel upon its application can be provided.

According to the present invention, there is thus provided a cosmetic composition comprising the following components (A), (B) and (C):
(A) a quaternary ammonium base-containing cationic crosslinked copolymer;
(B) powder having an average particle size of 0.05 to 50 µm; and
(C) a water-compatible solvent.

### BEST MODE FOR CARRYING OUT THE INVENTION

The copolymer of the component (A) useful in the present invention is a quaternary ammonium base-containing cationic crosslinked copolymer (hereinafter referred to as "Copolymer (A)). The fact that this copolymer contains a quaternary ammonium base and is used in combination with the powder (B) having the specific particle size and the water-compatible solvent (C) brings about the effects of the present invention. Japanese Patent Application Laid-Open Nos. 268857/1996 and 208452/1997 disclose cosmetic compositions in which a cationic thickener obtained by polymerizing a monomer having a tertiary amino group is incorporated. However, such a tertiary amino group-containing polymer or an acid-addition salt thereof has difficulty in bringing about the good dispersibility of powder and good feel upon application like the present invention.

No particular limitation is imposed on Copolymer (A) so far as it has both quaternary ammonium base and crosslinked structure in its molecule, and examples thereof include quaternary ammonium base-containing cationic crosslinked copolymers obtained by providing, as constituent monomers, at least one quaternary ammonium base-containing vinyl monomer (hereinafter referred to as "Monomer (a₁)"), at least one amide group-containing vinyl monomer (hereinafter referred to as "Monomer (a₂)) and at least one crosslinkable monomer (hereinafter referred to as Monomer (a₃)) having 2 or more vinyl groups in its molecule and subjecting these monomers to radical polymerization.

Examples of Monomer (a₁) among the constituent monomers of Copolymer (A) include quaternary ammonium salts of tertiary amino group-containing monomers such as (meth)acrylic esters and (meth)acrylamides having a dialkylamino group, styrene having a dialkyl amino group and derivatives thereof, vinylpyridine and derivatives thereof, N-vinyl heterocyclic compounds, and aminoalkyl vinyl ethers, and diallyl type quaternary ammonium salts.

Preferable specific examples of Monomer (a₁) include quaternary ammonium salts obtained by guaternizing a dialkylaminoalkyl (meth)acrylate or dialkylaminoalkyl(meth)acrylamide such as dimethylaminoethyl (meth)acrylate, diethylaminoethyl (meth)acrylate, dimethylaminopropyl(meth)acrylamide or diethylaminopropyl(meth)acrylamide with a quaternizing agent, and dimethyldiallylammonium chloride.

Preferable examples of the quaternizing agent used for obtaining the quaternary ammonium salts include alkyl halides such as methyl chloride and methyl iodide, and dialkyl sulfates such as diethyl sulfate and di-n-propyl sulfate.

Examples of Monomer (a₂) include those having an amide group and containing no quaternary ammonium base, for example, monomers represented by the general formulae (1) and (2): 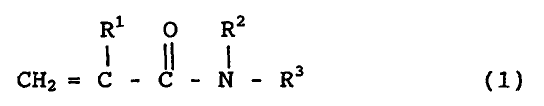 wherein R¹ is a hydrogen atom or methyl group, and R² and R³ are the same or different from each other and individually a hydrogen atom or a linear or branched alkyl group or alkenyl group having 1 to 4 carbon atoms, or form together -(CH₂)ₙ- (n being an integer of 3 to 6) or -(CH₂)₂O-(CH₂)₂- to form a ring containing the adjacent nitrogen atom; and 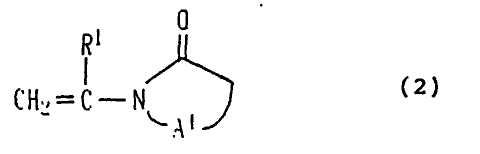 wherein R¹ has the same meaning as defined above, and A¹ is -(CH₂)ₘ- (m being an integer of 2 to 5). Specific examples thereof include N,N-dialkyl(meth)acrylamides such as N,N-dimethyl(meth)acrylamide and N,N-diethyl(meth)acrylamide; N-alkyl(meth)acrylamides such as N-methyl(meth)acrylamide, N-n-propyl(meth)acrylamide and N-t-butyl(meth)acrylamide; and N-(meth)acryloylmorpholine, N-vinylpiperidone and N-vinylpyrrolidone. Among these, N,N-dialkyl(meth)acrylamides are preferred from the viewpoint of a feel upon use, with N,N-dimethyl(meth)acrylamide and N,N-diethyl(meth)acrylamide being particularly preferred.

Examples of Monomer (a₃) include poly(meth)acrylic esters of polyhydric alcohols, (meth)acrylic esters of unsaturated alcohols, diacrylamide, divinyl compounds and polyallyl compounds. Among these, ethylene glycol di(meth)acrylate, polyethylene glycol di(meth)acrylate, pentaerythritol tetra(meth)acrylate, allyl etherified product of pentaerythritol, vinyl (meth)acrylate, allyl (meth)acrylate, etc. are particularly preferred.

A preferable blending ratio of Monomer (a₁) to Monomer (a₂) is 2/98 to 98/2, more preferably 3/97 to 60/40 in terms of a molar ratio of (a₁)/(a₂). Although development of thixotropic property is made easy when the molar ratio is lower, while the retention of viscosity at a low shear rate is made easy when the molar ratio is higher, It is preferred that the molar ratio should fall within the above range in order to exhibit both properties.
A proportion of Monomer (a₃) is preferably 0.002 to 5 % by weight, particularly 0.002 to 3 % by weight, more particularly 0.002 to 1 % by weight based on the total weight of the monomers. When the proportion of Monomer (a₃) falls within the above range, the viscosity of hydrogel formed from the resulting Copolymer (A) is sufficient, and the feel of the hydrogel is soft and smooth.

In addition to each at least one of the above-described three kinds of vinyl monomers, Copolymer (A) may contain other vinyl monomers copolymerizable therewith as a constituent component. Examples of said other vinyl monomers include (meth)acrylic esters such as methyl (meth)acrylate, ethyl (meth)acrylate; anionic group-containing monomers such as acrylic acid and methacrylic acid; and betaines such as N-(3-sulfopropyl)- N-acryloyloxyethyl-N,N-dimethylammonium betaine and N-carboxymethyl-N-methacryloyloxyethyl-N,N-dimethylammonium betaine.

Copolymer (A) can be prepared by means of an aqueous solution polymerization process, reversed phase suspension polymerization process, precipitation polymerization process or the like in accordance with a method known *per se* in the art.

The component (A) may be either a single copolymer or a mixture of copolymers. It is preferably incorporated in a proportion of 0.01 to 20 % by weight, particularly 0.02 to 10 % by weight, more particularly 0.1 to 5 % by weight based on the total weight of the composition from the viewpoints of dispersibility of powder and a feel.

No particular limitation is imposed on the powder of the component (B) useful in the present invention so far as it is that commonly used in conventional cosmetic compositions, and examples thereof include organic powders such as fine polymer particles obtained by conducting dispersion polymerization of a vinyl monomer in a solvent using, as a dispersing agent, a polysiloxane compound having a radical-polymerizable group at one terminal thereof (hereinafter referred to as "Polymer Beads S"), silicone resins (KMP-590 (product of Shin-Etsu Chemical Co., Ltd.), Tospearl 145 and Tospearl 2000 B (products of Toshiba Silicone Co., Ltd.), Trefil (product of Toray Industries, Inc.), etc.), nylon resins (SP-500 (product of Toray Industries, Inc.), etc.), polystyrene resins (Fine Pearl (product of Sumitomo Chemical Co., Ltd.), Techpolymer SB (product of Sekisui Plastics Co., Ltd.), Fine Powder SGP (product of Soken Chemical & Engineering Co., Ltd.), etc.), polyethylene resins (Flow Beads (product of Sumitomo Seika Co., Ltd.), etc.), polymethyl methacrylate resins (Matsumoto Microsphere M (product of Matsumoto Yushi-Seiyaku Co., Ltd.), Techpolymer MB (product of Sekisui Plastics Co., Ltd.), Fine Powder MP (product of Soken Chemical & Engineering Co., Ltd.), etc.), divinylbenzene resins, synthetic silica beads, polyurethane resins, benzoguanamine resins, melamine resins, phenol resins, and fluororesins; and inorganic powders such as talc, sericite, mica, kaolin, red iron oxide, clay, bentonite, silicic acid, silicic anhydride, magnesium silicate, magnesium oxide, zinc oxide, titanium oxide, aluminum oxide, aluminum sulfate, alum, calcium sulfate, barium sulfate and magnesium sulfate.

The form of the powder may be any form of spherical, columnar, plate and needle forms and vary as necessary for the end application intended. When the powder is used in pre-shave cosmetic compositions and deodorant compositions, a spherical form is preferred from the viewpoints of a feel and sliding. No particular limitation is imposed on the sphericity of the powder. Since the coefficient of dynamic friction of the powder lowers as the sphericity increases, and so a feeling of smooth sliding on the skin is enhanced, it is preferred to use powder near a true sphere to the extent possible.

The average particle size of the powder is 0.05 to 50 µm, preferably 0.5 to 50 µm. The average particle size within this range is preferred from the viewpoints of a feel to the skin and lubricating ability when the powder is used in pre-shave cosmetic compositions and deodorant compositions. It is particularly preferred that powder having a particle size of 1 to 20 µm be contained in a proportion of at least 80 % by weight based on the whole powder. It is desirable that any powder having a particle size of 200 µm or more is substantially not contained.

These powders may be subjected to a hydrophobicity-imparting treatment such as a treatment with a silicone, a fluorination, metal soap, a treatment with fatty acid or the like.

The component (B) may be either a single powder or a mixture of different powders. It is preferably incorporated in a proportion of 0.1 to 10 % by weight, particularly 1 to 7 % by weight, more particularly 2 to 6 % by weight based on the total weight of the composition in that sufficient effects are achieved, and the skin applied by the resulting cosmetic composition does not become whitish.

No particular limitation is imposed on the water-compatible solvent of the component (C) useful in the present invention so far as it has a solubility of at least 5 in water at 20°C, and examples thereof include lower alcohols having 1 to 4 carbon atoms, polyhydric alcohols and glycol ethers.

Examples of the lower alcohols include ethanol and isopropanol, and examples of polyhydric alcohols include ethylene glycol, propylene glycol, isoprene glycol, hexylene glycol, 1,3-butylene glycol, diethylene glycol, dipropylene glycol, glycerol, pentaerythritol and sorbitol.

Examples of the glycol ethers include ethylene glycol monoethyl ether, diethylene glycol monomethyl ether, diethylene glycol monoethyl ether and propylene glycol monoethyl ether.

As the water-compatible solvent of the component (C), a lower alcohol or polyhydric alcohol is preferred, with ethanol or glycerol being particularly preferred.

The component (C) may be either a single solvent or a mixture of solvents. It is preferably incorporated in a proportion of 0.01 to 95 % by weight, particularly 0.1 to 65 % by weight, more particularly 1 to 60 % by weight based on the total weight of the composition from the viewpoints of solubility and stability.

It is preferred to use ethanol as the component (C) in the case of a pre-shave cosmetic composition or deodorant composition. Ethanol is incorporated in a proportion of at least 40 % by weight, preferably at least 50 % by weight, more preferably 60 to 95 % by weight, particularly preferably 65 to 90 % by weight.

A germicide and/or an antiperspirant may be incorporated into the cosmetic composition according to the present invention. When these components are incorporated, a deodorant composition which can sustain the deodorant effect over a long period of time by the action of Copolymer (A) while retaining good dispersibility of the powder and a dry feeling is provided. Examples of the germicide include 3,4,4-trichlorocarbanilide (TCC), benzethonium chloride, benzalkonium chloride, alkyltrimethylammonium chlorides, resorcin, phenol, sorbic acid, salicylic acid, hexachlorophene, triclosan, isopropylmethylphenol and zinc pyrithione. Examples of the antiperspirant include aluminum hydroxy chloride, aluminum chloride, aluminum sulfate, basic aluminum bromide, aluminum phenolsulfonate, tannic acid, aluminum naphthalenesulfonate, basic aluminum iodide, zirconium salts, aluminum-zirconium complexes and zinc p-phenolsulfonate. The germicide is preferably incorporated in a proportion of 0.005 to 5.0 % by weight, particularly 0.01 to 2.0 % by weight based on the total weight of the composition, while the antiperspirant is preferably incorporated in a proportion of 0.1 to 30 % by weight, particularly 1.0 to 15.0 % by weight based on the total weight of the composition.

In the cosmetic composition according to the present invention, anionic surfactants, amphoteric surfactants, nonionic surfactants, cationic surfactants, anionic polymers, nonionic polymers, cationic polymers, ethylene oxide-propylene oxide block copolymers, oily substances, functional beads or capsules, silicones, metal chelating agents, antioxidants, ultraviolet absorbents, moisturizers, anti-inflammatory agents, astringents, colorants, perfume bases, water, etc., which are commonly used in conventional cosmetic compositions, may be suitably incorporated in addition to the above-described components.

The cosmetic composition according to the present invention can be prepared in accordance with a method known *per se* in the art without any particular limitation to the forms thereof, and may be applied as, for example, hair cosmetic compositions such as hair liquids, hair growth stimulants, hair styling compositions and hair treatments; and skin cosmetic compositions such as cosmetic lotions, emulsified cosmetic compositions, deodorants, shaving gel, pre-shave lotions, after-shave lotions and liquid foundations.

In the present invention, the respective components to be incorporated can be selected to provide a cosmetic composition in which the powder is uniformly dispersed, or a cosmetic composition of a two-layer separation type in which the powder precipitates when left at rest. In the case of the two-layer separation type, the powder is uniformly dispersed by shaking before use. In this case, the powder can be uniformly dispersed again with ease.

### EXAMPLES

### Preparation Example 1:

A reaction vessel was charged with an aqueous monomer solution composed of N,N-dimethylaminoethyl methacrylate quaternized with diethyl sulfate (MOEDES; product of Nitto Chemical Industry Co., Ltd.; 23.85 g), N,N- dimethylacrylamide (71.37g), polyethylene glycol dimethacrylate (NK-9G; product of Shin-Nakamura Chemical Co., Ltd.; 0.0429 g) and ion-exchanged water (350 g) and purged with nitrogen in advance, and nitrogen was additionally blown for 20 minutes into the reaction vessel, thereby heating the contents to 55°C while purging the reaction system with nitrogen. A polymerization initiator (2,2'-azobis(2-amidinopropane) dihydrochloride; 0.22 g) was then added. The monomer solution began to polymerize after 30 minutes to 1 hour to form soft gel as a whole. Stirring was continued as it is to stop the polymerization after 4 hours from the addition of the polymerization initiator. The contents in the form of a rice cake were taken out of the reaction vessel, washed 10 minutes in ethanol (5 liters) under stirring and dried. The dry product was then ground by means of a coffee mill and a jet mill, and the ground particles were classified by a high bolter to obtain Cationic Crosslinked Copolymer No. 1.

### Preparation Examples 2 to 18:

Their corresponding monomers shown in Table 1 were used to prepare Cationic Crosslinked Copolymer Nos. 2 to 18 in a similar manner to Example 1.

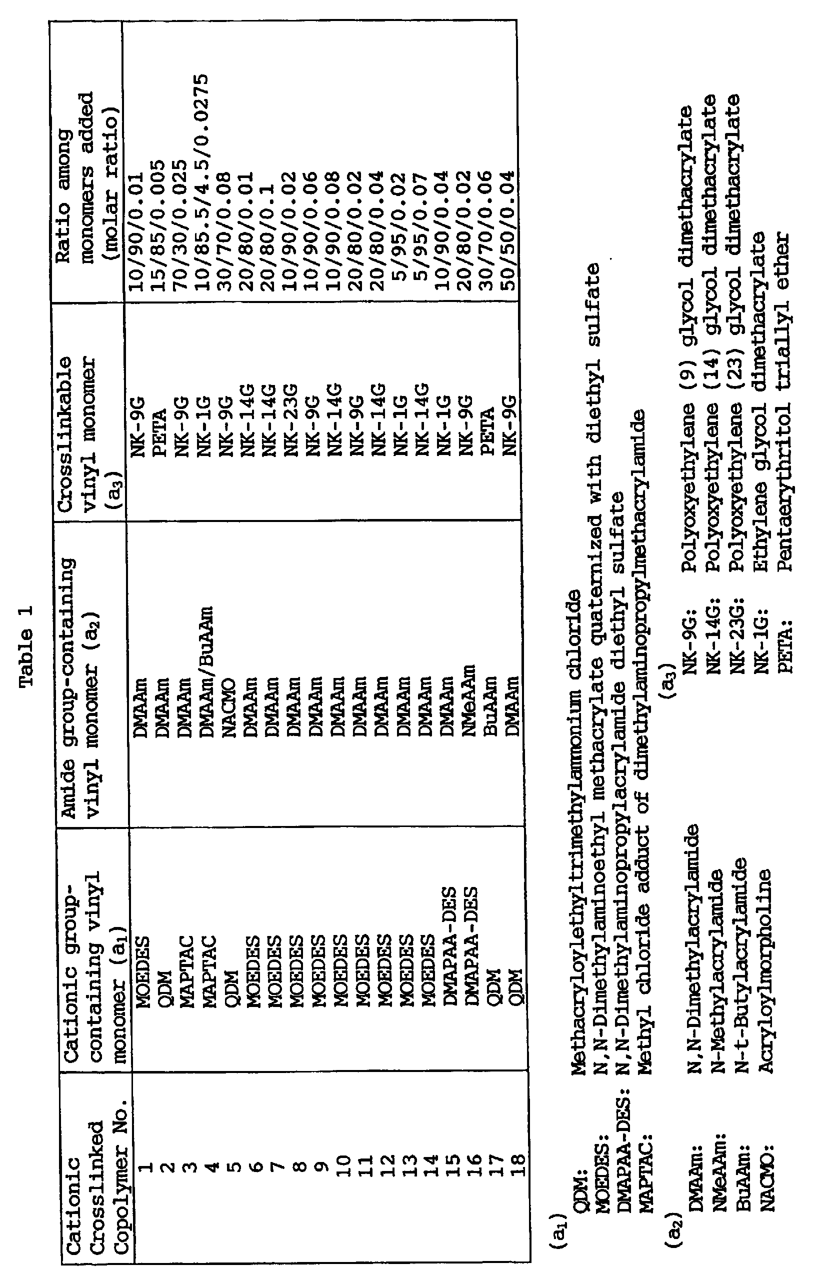

### Example 1:

Pre-shave cosmetic compositions of their corresponding formulations shown in Table 2 were prepared in accordance with a method known *per se* in the art to evaluate them as to redispersibility of powder, easy application to the skin, sliding of shaver and a feel to the skin after shaving. The results are shown collectively in Table 2.

### (Evaluating Method)

A pre-save cosmetic composition sample was applied in a proper amount to the skin to shave by means of an electric razor. At every stage, the evaluation was organoleptically conducted by 15 expert panelists to rank the sample in accordance with the following respective standards. Respective average scores were found to express the results in accordance with the following criterion.

### (1) Redispersibility of powder:

| | |
|---|---|
| Very good | Score 4 |
| Good | Score 3 |
| Somewhat poor | Score 2 |
| Poor | Score 1 |

### (2) Easy application to the skin:

| | |
|---|---|
| Very easy to apply | Score 4 |
| Easy to apply | Score 3 |
| Somewhat hard to apply | Score 2 |
| Hard to apply | Score 1 |

### (3) Sliding of shaver:

| | |
|---|---|
| Very smoothly slid | Score 4 |
| Smoothly slid | Score 3 |
| Not very smoothly slid | Score 2 |
| Not smoothly slid | Score 1 |

### (4) Feel to the skin after shaving:

| | |
|---|---|
| Very good | Score 4 |
| Good | Score 3 |
| Not very good | Score 2 |
| Felt sticky | Score 1 |

### 〈Criteria〉

| | |
|---|---|
| Average score 3.5 to 4.0 | ⓞ |
| Average score 2.5 to 3.4 | ○ |
| Average score 1.5 to 2.4 | △ |
| Average score 1.0 to 1.4 | X |

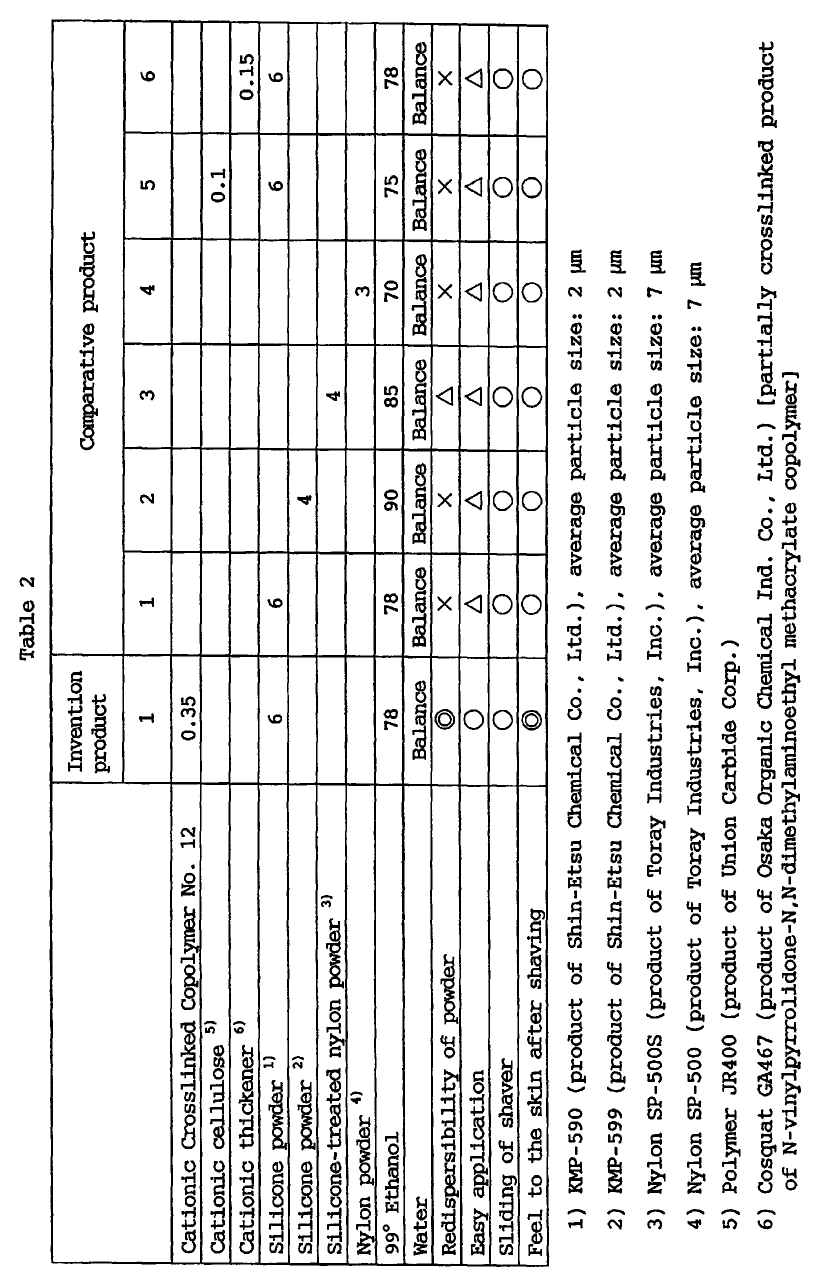

### Examples 2 to 10:

Pre-shave cosmetic compositions of their corresponding formulations shown in Table 3 were prepared in accordance with a method known *per se* in the art.

All the pre-shave cosmetic compositions thus obtained were easy to apply to the skin, had a good feel to the skin and were high in shaving effect and excellent in redispersibility of powder.

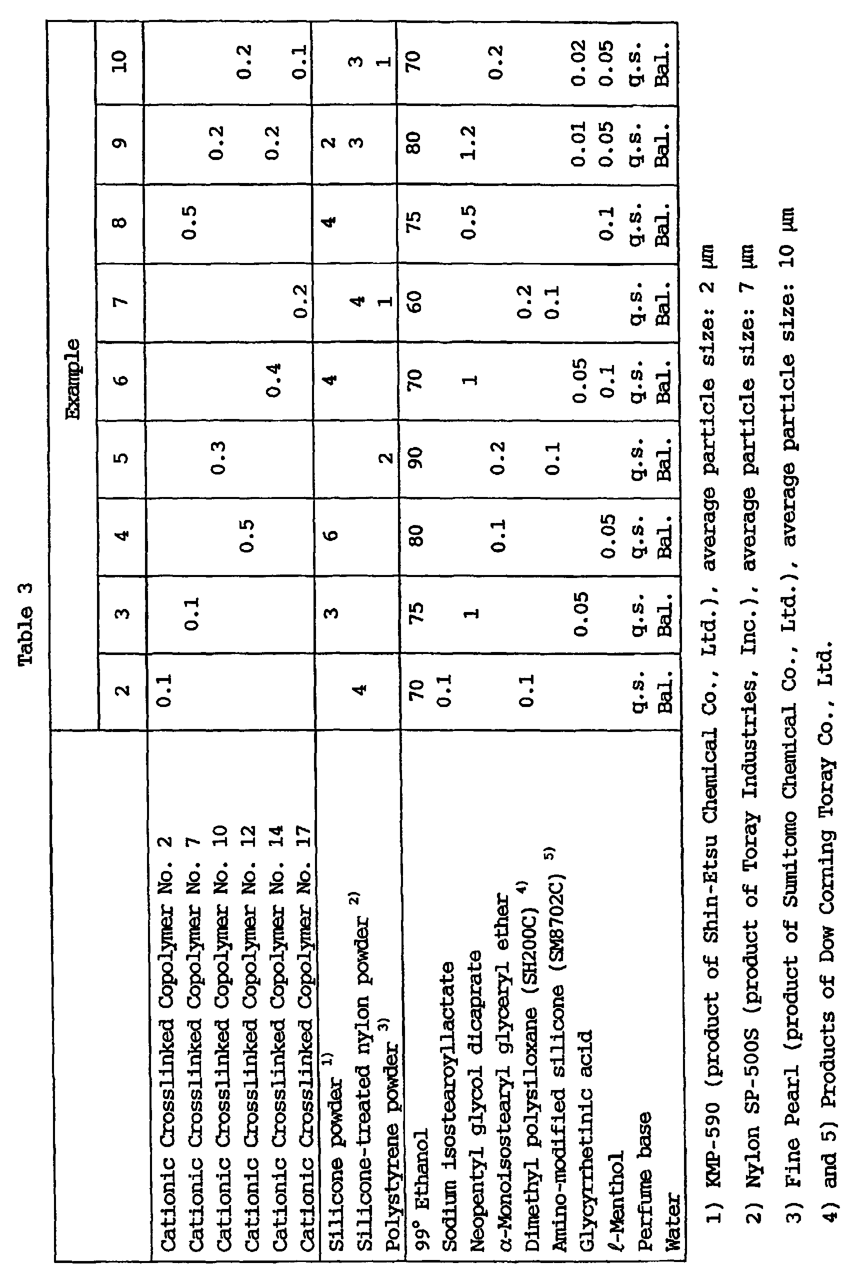

### Example 11:

Cosmetic compositions of their corresponding formulations shown in Table 4 were prepared in accordance with a method known *per se* in the art to evaluate them as to dispersibility of powder (visual observation; redispersibility in the case of a two-layer separation type; evaluation standard is the same as in Example 1), and a feel upon application to the hair and skin and a feel after drying. The results are shown collectively in Table 4.

### (Evaluating Method)

The evaluation to the hair was conducted by washing a hair tress having a length of 20 cm and a weight of 30 g with a shampoo, applying a cosmetic composition sample (1 g) thereto and then drying the hair. The evaluation to the skin was conducted after the cosmetic composition sample (1 g) was applied to an inner arm and air-drying it for 30 minutes. The feel at every stage was organoleptically evaluated by 15 expert panelists to rank the sample in accordance with the following respective standards. Respective average scores were found to express the results in accordance with the following criterion. (1) Feel upon application to the hair:

| | |
|---|---|
| Gave a very good feel to the hair upon application | Score 4 |
| Gave a good feel to the hair upon application | Score 3 |
| Gave a somewhat poor feel to the hair upon application | Score 2 |
| Gave a poor feel to the hair upon application | Score 1 |

### (2) Feel after drying the hair:

| | |
|---|---|
| Hair was very smooth after drying | Score 4 |
| Hair was smooth after drying | Score 3 |
| Hair was not very smooth after drying | Score 2 |
| Hair was not smooth after drying | Score 4 |

### (3) Feel upon application to the skin:

| | |
|---|---|
| Sliding was very good | Score 4 |
| Sliding was good | Score 3 |
| Sliding was not very good | Score 2 |
| Sliding was not good | Score 1 |

### (4) Feel after drying the skin:

| | |
|---|---|
| Sliding was very good | Score 4 |
| Sliding was good | Score 3 |
| Sliding was not very good | Score 2 |
| Felt sticky | Score 1 |

### 〈Criteria〉

| | |
|---|---|
| Average score 3.5 to 4.0 | ⓞ |
| Average score 2.5 to 3.4 | ○ |
| Average score 1.5 to 2.4 | △ |
| Average score 1.0 to 1.4 | X |

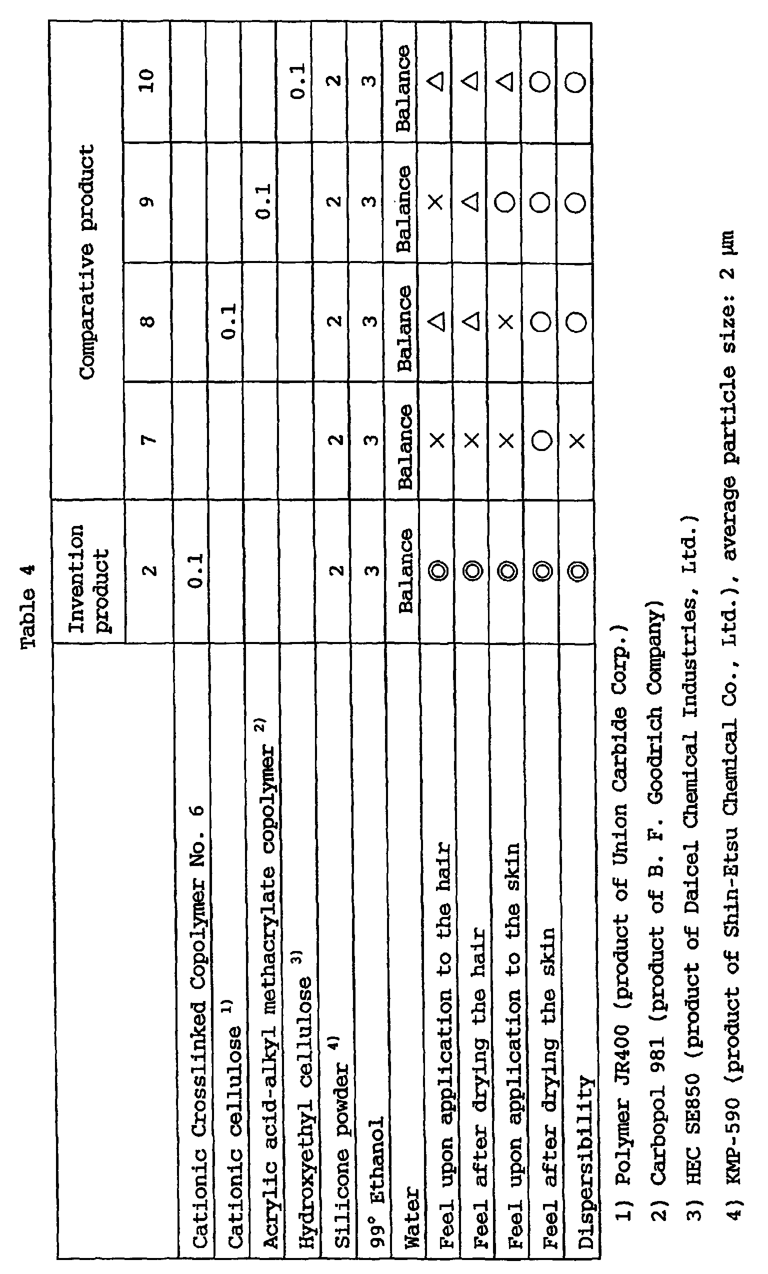

### Examples 12 to 20:

Cosmetic compositions of their corresponding formulations shown in Table 5 were prepared. All these cosmetic compositions were good in dispersibility of powder and excellent in a feel upon application to the hair and skin, and gave users a smooth feeling after drying.

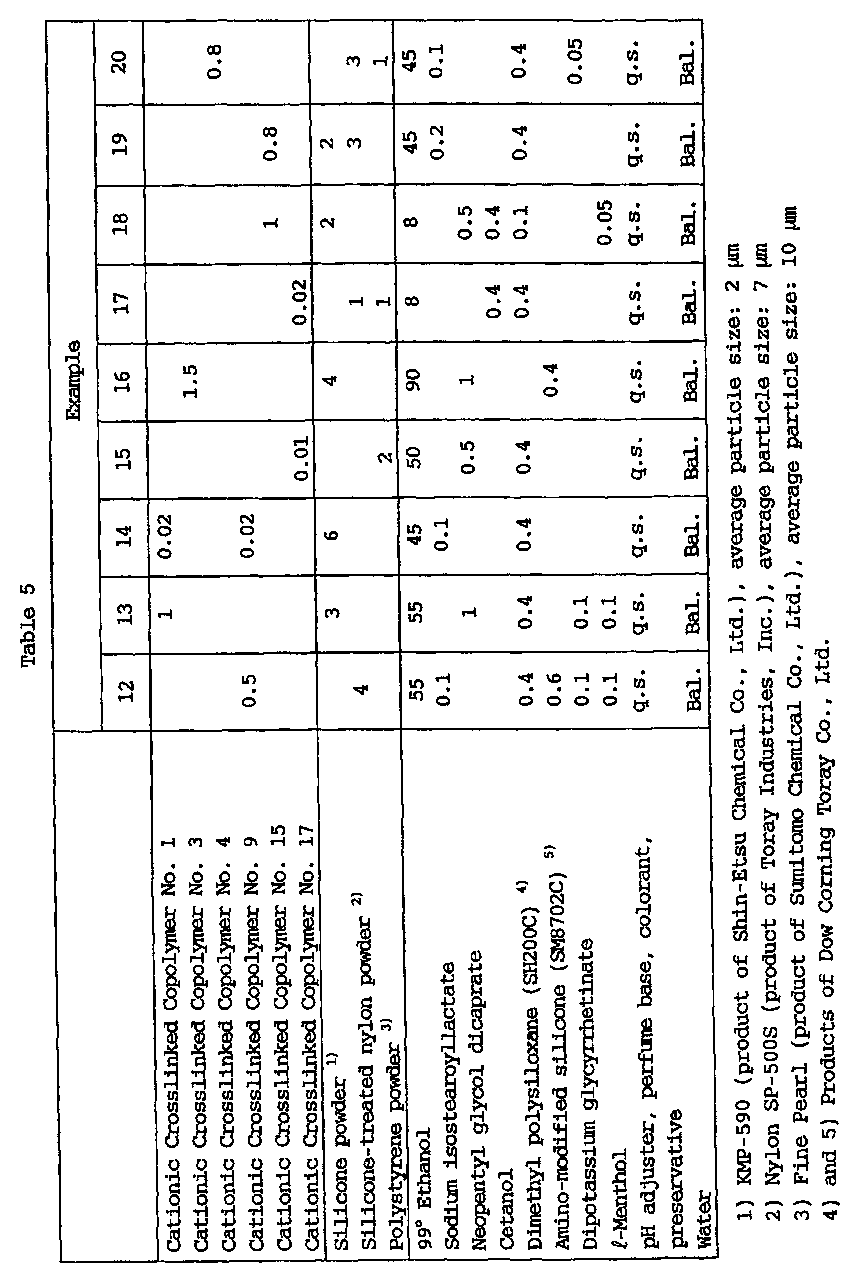

### Example 21:

Deodorant compositions of their corresponding formulations shown in Table 6 were prepared in accordance with a method known *per se* in the art to evaluate them as to redispersibility of powder (visual observation; evaluation standard is the same as in Example 1), a dried feeling to the skin and deodorant effect. The results are shown collectively in Table 6.

### (Evaluating Method)

A deodorant composition sample was applied in a proper amount to a part that odor weighs on one's mind to organoleptically evaluate the sample. The evaluation was conducted by 15 expert panelists to rank the sample in accordance with the following respective standards. Respective average scores were found to express the results in accordance with the following criterion.

### (1) Dried feeling to the skin:

| | |
|---|---|
| Very good | Score 4 |
| Good | Score 3 |
| Somewhat poor | Score 2 |
| Poor | Score 1 |

### (2) Deodorant effect:

| | |
|---|---|
| Very high in deodorant effect | Score 4 |
| High in deodorant effect | Score 3 |
| Somewhat high in deodorant effect | Score 2 |
| Low in deodorant effect | Score 1 |

### (3) Redispersibility of powder:

| | |
|---|---|
| Very good | Score 4 |
| Good | Score 3 |
| Somewhat poor | Score 2 |
| Poor | Score 1 |

### 〈Criteria〉

| | |
|---|---|
| Average score 3.5 to 4.0 | ⓞ |
| Average score 2.5 to 3.4 | ○ |
| Average score 1.5 to 2.4 | △ |
| Average score 1.0 to 1.4 | X |

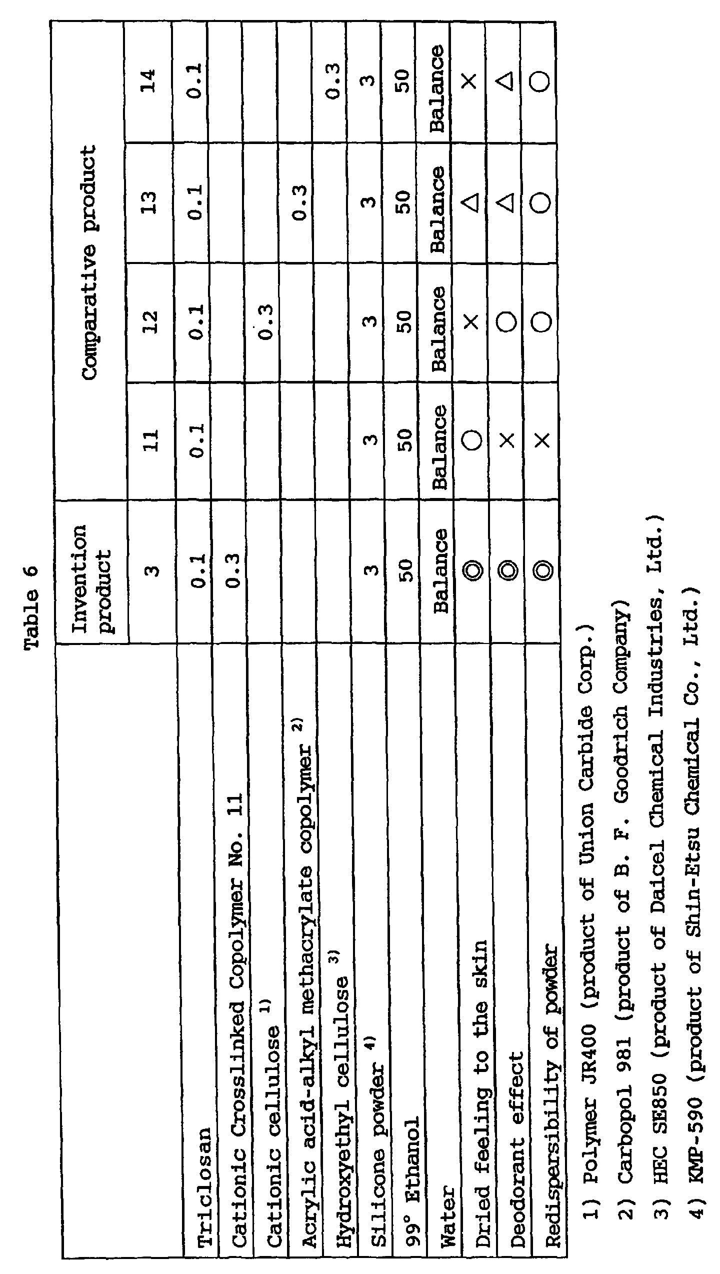

### Examples 22 to 27

Deodorant compositions of their corresponding formulations shown in Table 7 were prepared. All these deodorant compositions were good in redispersibility of powder and excellent in dried feeling to the skin and deodorant effect.

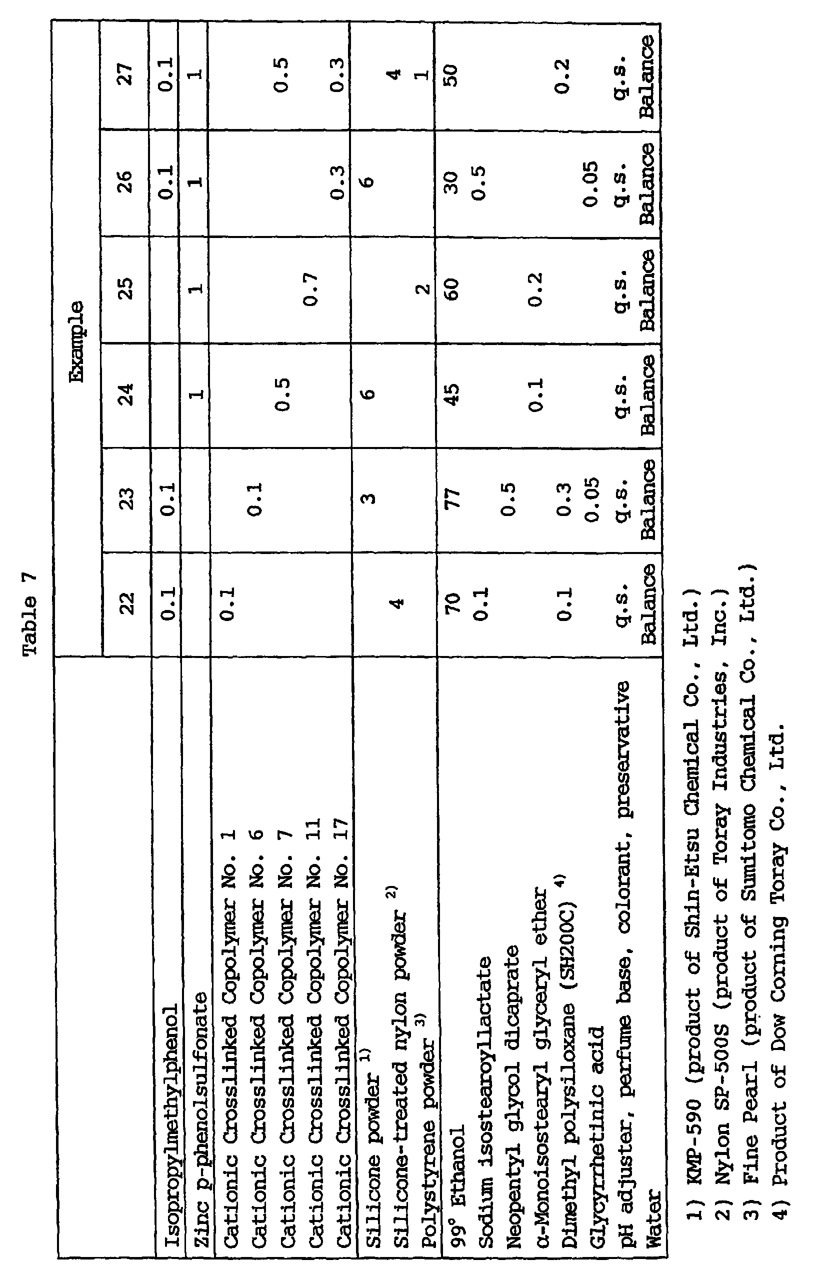

### INDUSTRIAL APPLICABILITY

The cosmetic compositions according to the present invention are good in dispersibility of powder, easy to apply to the skin and also good in a feel upon application and after drying. When applied as pre-shave cosmetic compositions, shaving effects such as good sliding of a shaver are high. When applied as two-layer separation type compositions, redispersibility of powder is excellent.

## Claims

1. A cosmetic composition comprising the following components (A), (B) and (C):
(A) a quaternary ammonium base-containing cationic crosslinked copolymer;
(B) powder having an average particle size of 0.05 to 50 µm; and
(C) a water-compatible solvent.

2. The cosmetic composition according to Claim 1, wherein the component (A) is a quaternary ammonium base-containing cationic crosslinked copolymer obtained by providing, as constituent monomers, at least one quaternary ammonium base-containing vinyl monomer, at least one amide group-containing vinyl monomer and at least one crosslinkable monomer having 2 or more vinyl groups in its molecule and subjecting the monomers to radical polymerization.

3. The cosmetic composition according to Claim 2, wherein the amide group-containing vinyl monomer is a monomer represented by the general formula (1) or (2): 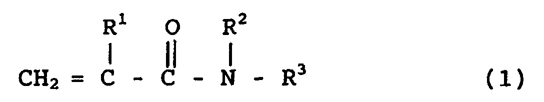 wherein R¹ is a hydrogen atom or methyl group, and R² and R³ are the same or different from each other and individually a hydrogen atom or a linear or branched alkyl group or alkenyl group having 1 to 4 carbon atoms, or form together -(CH₂)ₙ- (n being an integer of 3 to 6) or -(CH₂)₂O-(CH₂)₂- to form a ring containing the adjacent nitrogen atom; or 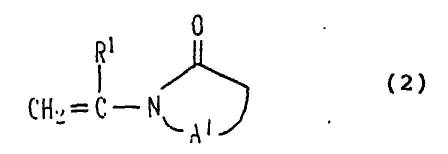 wherein R¹ has the same meaning as defined above, and A¹ is -(CH₂)ₘ- (m being an integer of 2 to 5).

4. The cosmetic composition according to any one of Claims 1 to 3, which further comprises a germicide and/or an antiperspirant.

5. The cosmetic composition according to any one of Claims 1 to 4, wherein the cosmetic composition is a skin cosmetic composition or hair cosmetic composition.
